(19) **Europäisches Patentamt**
**European Patent Office**
**Office européen des brevets**

(11) Veröffentlichungsnummer: **0 545 174 B1**

(12) **EUROPÄISCHE PATENTSCHRIFT**

(45) Veröffentlichungstag der Patentschrift: **13.09.95**

(51) Int. Cl.6: **C07C 17/26**, C07C 43/17, C09G 3/00

(21) Anmeldenummer: **92119787.7**

(22) Anmeldetag: **20.11.92**

(54) **Oligomere von fluorierten Olefinen.**

(30) Priorität: **03.12.91 DE 4139765**

(43) Veröffentlichungstag der Anmeldung:
**09.06.93 Patentblatt 93/23**

(45) Bekanntmachung des Hinweises auf die Patenterteilung:
**13.09.95 Patentblatt 95/37**

(84) Benannte Vertragsstaaten:
**AT CH DE FR GB IT LI**

(56) Entgegenhaltungen:
**DE-A- 4 000 661**

**CHEMICAL ABSTRACTS, vol. 66, no. 21, 22. Mai 1967, Columbus, Ohio, US; abstract no. 94680j, B. I. KEDA ET AL. 'Telomerization of allyl acetate by cyanogen chloride and oligomerization of 3,3,3-trifluoropropene in the presence of free radical initiators' Seite 8847 ;Spalte 2 ;**

**JOURNAL OF FLUORINE CHEMISTRY Bd. 48, Nr. 2, 15. Juni 1990, LAUSANNE CH Seiten 229 - 237 W. KEIM ET AL. 'Transition Metal Catalysed C-C-Coupling Reactions of 3,3,3-Trifluoropropene'**

(73) Patentinhaber: **HOECHST AKTIENGESELL-SCHAFT**

**D-65926 Frankfurt am Main (DE)**

(72) Erfinder: **Von Werner, Konrad, Dr. Wehrstrasse 6 W-8268 Garching (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 545 174 B1

**Beschreibung**

Die Erfindung betrifft Oligomere von Verbindungen der Formel

$$X\text{-}(CF_2)_a\text{-}O_b\text{-}(CH_2)_c\text{-}CH = CH_2 \qquad (1)$$

sowie Co-Oligomere aus Verbindungen der Formel (1) mit Verbindungen der Formel

$$X\text{-}(CX_2)_d\text{-}O_b\text{-}(CX_2)_c\text{-}CX = CX_2 \qquad (2) .$$

In diesen Formeln bedeuten:

X = Wasserstoff oder Fluor,

a = eine Zahl von 2 bis 16,

b und c sind - unabhängig voneinander - 0 oder 1 und

d = eine Zahl von 0 bis 6.

Bevorzugt sind Oligomere, in denen X Fluor, a eine Zahl von 4 bis 12 sowie b und c Null bedeuten.

Der mittlere Oligomerisationsgrad dieser Verbindungen ist 2 bis 4.

Die erfindungsgemäßen Oligomeren erhält man durch Erhitzen einer Lösung von mindestens einer Verbindung der Formel (1), gegebenenfalls mit mindestens einer Verbindung der Formel (2), in einem Kohlenwasserstoff auf eine Temperatur von 135 bis 180 °C mit einem radikalbildenden Katalysator.

Bevorzugt sind gesättigte Kohlenwasserstoffe als Lösemittel wie Alkane mit 5 bis 12 Kohlenstoffatomen und leicht zugängliche Cycloalkane wie Cyclohexan.

Als radikalbildende Katalysatoren werden Peroxide, vor allem Dialkylperoxide, bevorzugt. Vor allem kommen symmetrische Dialkylperoxide mit jeweils 6 bis 16 C-Atomen in Betracht.

Die bevorzugte Reaktionstemperatur liegt im Bereich von 140 bis 160 °C.

Bevorzugte Ausgangsmaterialien der Formel (1) sind Perfluoralkylethylene mit einem Perfluoralkylrest mit 4 bis 12 Kohlenstoffatomen sowie die entsprechenden Verbindungen mit einem terminalen Wasserstoffatom an der Fluoralkankette. Weiterhin bevorzugt sind die entsprechenden Vinyl- und Allylether.

Bevorzugte Comonomere der Formel (2) sind gegebenenfalls fluorierte mono-ethylenisch ungesättigte Kohlenwasserstoffe mit 2 bis 6 Kohlenstoffatomen sowie gegebenenfalls fluorierte Alkylvinyl- und -allylether. Genannt seien Ethylen, 1-Octen, 1,1,2,2-Tetrafluorethyl-3',3'-difluorallylether sowie Hexafluorpropen.

Die erfindungsgemäßen Oligomeren und Co-Oligomeren sind stark hydrophob und relativ oleophob, aber fluorophil. Sie eignen sich deshalb als Gleitmittel auf Oberflächen, insbesondere aus Kunststoff, Metall oder Glas. Die Produkte lassen sich beispielsweise leicht durch einfaches Verreiben auf Polyethylenfolien aufbringen. Auf so behandelten Folien läuft Wasser bei der leichtesten Erschütterung in Form fast kugelförmiger Tropfen herum, während es auf nicht behandelten Folien deutlich haftet. Werden die erfindungsgemäßen Verbindungen auf gegebenenfalls graphitierte Polyethylen-Skisohlen aufgebracht, so ergibt sich ein sehr gutes Laufverhalten auf feuchtem Schnee. Die Applikation kann in üblicher Weise erfolgen, beispielsweise als Skiwachs. Die erfindungsgemäßen Verbindungen lassen sich auch durch Preß-Sintertechnik zusammen mit Polyethylen zu einem Material verarbeiten, aus dem Skibeläge mit hoher Gleitfähigkeit hergestellt werden können.

Da sich die erfindungemäßen Oligomeren mit Perfluoralkanen mischen lassen, können mit ihrer Hilfe sonst schwer auf Flächen aufzubringende Fluoralkanwachse appliziert werden.

Hauchdünne Oligomerfilme erniedrigen die Glas-auf-Glas-Reibung extrem. Auch die Metall-Metall-Reibung wird stark verringert. Ein Zusatz von Polytetrafluorethylen-Mikropulver ergibt hierbei eine weitere Verbesserung.

Die erfindungemäßen Verbindungen eignen sich somit vorzüglich für Beschichtungen von Oberflächen der verschiedensten Art, wobei die Reibung auf derart beschichteten Oberflächen stark herabgesetzt wird.

In den folgenden Beispielen wird die Erfindung näher erläutert. Die Molekulargewichte wurden mit einem Dampfdruck-Osmometer der Firma Knauer bestimmt.

Beispiel 1

Ein elektrisch beheizter Schüttelautoklav mit 4,5 l Inhalt wird mit einer Lösung von 1730 g (5,0 mol) $C_6F_{13}CH = CH_2$ und 51,2 g (0,35 mol) Di-tert.-butyl-peroxid in 2,0 l n-Hexan befüllt. Man spült 10 min mit einem leichten $N_2$-Strom, preßt dann zur Druckprobe 10 bar $N_2$ auf und entspannt langsam. Der Autoklav wird dann unter Schütteln in 1,5 h auf 150 °C geheizt und 5 h bei dieser Temperatur geschüttelt. Nach Abkühlung auf 30 °C (externe Kühlung durch Blasen mit Kaltluft) wird der Autoklav entleert. Man erhält ein zweiphasiges Gemisch, wobei die Oberphase hauptsächlich aus Hexan besteht. Das Lösemittel wird am Rotationsverdampfer entfernt. Das verbleibende Rohprodukt wird zur Entfernung geringer mechanischer Verunreinigungen bei leichtem Unterdruck über eine Kutsche mit Papierfilter abfiltriert. Das Filtrat wird an einer Vigreux-Kolonne andestilliert. Zwischen 25 und 105 °C Kopftemperatur bei 20 mbar gehen insgesamt 133 g über. Das mittlere Molgewicht $\overline{M}$ beträgt 562 - das Destillat enthält also etwa 60 % Dimere. Als Rückstand werden 1593 g eines fast farblosen Öls erhalten (92,1 % Ausbeute). Die mittlere Molmasse beträgt 1070, liegt also etwas über der Molmasse des $C_6F_{13}CH=CH_2$-Trimers (1038).

Das oligomere Produkt ist gegenüber heißer Natronlauge sehr stabil. Nach dreistündigem Rühren unter Rückfluß mit 20%iger NaOH (mit und ohne Phasentransferkatalysator) wurden in der wäßrigen Phase nur Spuren von Fluorid gefunden.

Beispiel 2

Verfährt man gemäß Beispiel 1, setzt jedoch Cyclohexan als Lösemittel ein, so erhält man ein vergleichbares Produkt in 91 % Ausbeute.

Beispiele 3 bis 10

In der folgenden Tabelle 1 wird die Herstellung weiterer Oligomerer und in der Tabelle 2 die Herstellung von Co-Oligomerisaten beschrieben.

Die Beispiele 7 bis 10 wurden mit jeweils 100 ml Hexan als Lösemittel durchgeführt.

Tabelle 1

| Beispiel | Alken | | | DTBO a) | | n-Hexan | Temperatur | Dauer | Ausbeute | Merkmale des Produkts |
|---|---|---|---|---|---|---|---|---|---|---|
| | Art | g | mol | g | Mol-% | ml | °C | h | g | |
| 3 | $C_8F_{17}CH=CH_2$ | 133,8 | 0,3 | 3,07 | 7 | 120 | 150 | 5 | 108,0 | dickes Öl |
| 4 | $R_FCH=CH_2$ b) | 119,9 | 0,25 | 2,56 | 7 | 100 | 150 | 5 | 115,3 | teilweise fest c) |
| 5 | $R_FCH=CH_2$ b) | 1438,5 | 3,0 | 30,7 | 7 | 1200 | 150 | 5 | 1325 | teilweise fest c) |
| 6 | $C_8F_{17}CH_2CH=CH_2$ | 115 | 0,25 | 2,56 | 7 | 100 | 150 | 5 | 113,0 | gelbliches Öl |

a) Di-tert.-butyl-peroxid

b) technisches Produkt: $R_F$ = 4,3 % $C_6F_{13}$, 44,0 % $C_8F_{17}$, 39,1 % $C_{10}F_{21}$, 3,4 % $C_{12}F_{25}$ und längerkettig, Rest Verunreinigungen wie $R_FH$.

c) Oberhalb 40 °C homogene Flüssigkeit. DSC-Messungen unter autogenem Druck zeigen keine nennenswerte Zersetzung bis 400 °C.

4

Tabelle 2

| Beispiel | Alken I | | | Alken II | | | DTBO [a] g | Temperatur °C | Dauer h | Ausbeute g |
|---|---|---|---|---|---|---|---|---|---|---|
| | Art | g | mol | Art | g | mol | | | | |
| 7 | $C_6F_{13}CH{=}CH_2$ | 69,2 | 0,2 | $C_6H_{13}CH{=}CH_2$ | 22,4 | 0,2 | 2,34 | 150 | 5 | 82,9 |
| 8 | $C_6F_{13}CH{=}CH_2$ | 86,5 | 0,25 | $CH_2{=}CH_2$ | 14 | 0,5 | 2,56 | 150 | 5 | 97,9 |
| 9 | $C_6F_{13}CH{=}CH_2$ | 51,9 | 0,15 | $CH_2{=}CHCH_2OCF_2CF_2H$ | 15,8 | 0,1 | 2,56 | 150 | 5 | 68,0 |
| 10 | $C_6F_{13}CH{=}CH_2$ | 86,5 | 0,25 | $CF_2{=}CF{-}CF_3$ | 37,5 | 0,25 | 2,56 | 150 | 5 | 80,6 [b] |

a) Di-tert.-butyl-peroxid

b) Hexafluorpropen-Anteil: 27,0 Mol-%

Beispiel 11

In einen evakuierten 10-l-Rührautoklav wird eine Mischung aus 3,2 l eines höheren Alkangemisches (SHELLSOL 145/160) vom Siedebereich 145 bis 160 °C, 4088 g (etwa 8 mol) Perfluoralkylethylen und - darin gelöst - 82 g (0,56 mol) Di-tert.-butyl-peroxid eingesaugt. Das Perfluoralkylethylen hatte (nach gaschromatographischer Untersuchung) die folgende Zusammensetzung:

- $C_nF_{2n+1}{-}CH{=}CH_2$

| | | |
|---|---|---|
| n = 8 | 1,2 % | |
| n = 10 | 67,5 % | |
| n = 12 | 24,8 % | |
| n = 14 | 4,1 % | |
| n ≧ 16 | 0,4 % | 98 % |

- $C_nF_{2n+1}$-H: 0,3 %
- andere Verunreinigungen: 1,7 %

Iodgehalt: 390 ppm

Das Restvakuum wird mit Stickstoff aufgefüllt und der Autoklav mit 5 bar Stickstoff gespült. Der entspannte und verschlossene Autoklav wird dann innerhalb von 1 1/2 h auf 150 °C aufgeheizt und 5 h gerührt, wobei ein maximaler Druck von 6 bar gemessen wird. Man läßt dann auf etwa 80 °C abkühlen und entleert den Autoklav über ein Tauchrohr. Das flüssige, zweiphasige Reaktionsprodukt wird bei 20 mbar bis zu einer Sumpftemperatur von 150 °C andestilliert. Das übergehende Lösemittel enthält nur geringe Anteile an fluorierten Komponenten und kann ohne weitere Reinigung für einen weiteren Ansatz eingesetzt werden. Der flüssige Destillationsrückstand (4170 g) erstarrt zu einem gut mahlbaren Produkt, das auch nach längerer Lagerzeit noch rieselfähig ist. Schmelzbereich 70 bis 80 °C.

Setzt man ein Perfluoralkylethylen ein, das niedriger siedende Anteile enthält, so erhält man erstarrende Produkte mit niedrigerem Schmelzbereich, beispielsweise bei Einsatz von $C_nF_{2n+1}$-CH=CH$_2$

| | | | |
|---|---|---|---|
| n = 6 | 30,3 % | n = 12 | 16,4 % |
| n = 8 | 4,1 % | n = 14 | 4,7 % |
| n = 10 | 39,9 % | n ≧ 16 | 1,6 % |

Iodgehalt: 200 ppm
ein Produkt vom Schmelzbereich 40 bis 60 °C.

Beispiel 12

Das Oligomer gemäß Beispiel 5 wird durch "Spincasting" aus einer 1,1,2-Trifluor-trichlorethan (®Frigen 113)-Lösung auf ein sorgfältig gereinigtes Glasplättchen aufgebracht. Nach Abdunsten des Lösemittels wird zur Orientierung des Oberflächenfilms das Plättchen auf 40 °C erwärmt. Aus den durch Immersion mit n-Dodecan und n-Hexadecan bestimmten Kontaktwinkeln $\theta$ wird eine dispersive Oberflächenenergie von nur 9 mN/m berechnet (nach W.A. Zisman "Contact angle, wettability, and adhesion", Adv. in Chemistry Series, No. 43, Am. Chem. Soc., Washington DC, 1964).

Beispiel 13

5000 g hochmolekulares, mikrogranulares Polyethylen (®Hostalen GM 7250, Hoechst AG), 1250 g amorpher Kohlenstoff (Acetylenruß) und 100 g des Perfluoralkylethylen-Oligomeren nach Beispiel 5 werden anteilig vorgemischt. Die Mischung wird in einem Planetenmischer bei maximal 70 °C homogenisiert und dann in einer beheizbaren Preßform unter allmählichem Aufheizen bis 200 °C und einem Druck von maximal 200 bar isostatisch zu einem Zylinder verpreßt und gesintert. Nach Abkühlen wird aus dem so erhaltenen Formkörper eine 1,5 mm dicke, nahezu schwarze Schälfolie hergestellt. Diese Folie wird poliert und weist nun eine glatte, rißfreie Oberfläche auf, wie sie für einen erstklassigen Rennski-Belag gefordert wird.

Gegenüber einer auf gleiche Weise ohne Zusatz des Oligomeren hergestellten Schälfolie weist die erfindungsgemäße Folie eine deutlich bessere Wasserabweisung auf. Untersuchungen mit Rasterelektronen-Mikroskop und ESCA (Electron Spectroscopy for Chemical Analysis) zeigen, daß das Oligomere vor allem in den Oberflächenbereichen angereichert ist, die aus amorphem Polyethylen bestehen.

Beispiel 14

Hochmolekulares Polyethylen (Hostalen GM 5010 T2) wird mit 0,5 Gew.-% erfindungsgemäßem Oligomer vermischt und auf einem Extruder zu Rohren mit einem Durchmesser von 32 mm und einer Wandstärke von 3 mm extrudiert. Bei einer Schneckendrehzahl von 80 Upm und einer Massetemperatur in der Mitte von 223 ± 1 °C und einer Massetemperatur außen von 211 ± 2 °C betrug der Massedruck 120 bar.

Bei einem Kontrollansatz ohne Oligomer-Zusatz stellte sich ein Massedruck von 135 bar ein.

Gegenüber dem ohne Oligomer-Zusatz erhaltenen Rohr zeigten die mit Oligomer-Zusatz extrudierten Rohre einen stark erhöhten Glanz und beim Durchfluß eines zähflüssigen Öls eine sehr viel geringere Haftung an der Rohrwandung.

Es wurden die folgenden Oligomeren eingesetzt:
- Versuch 1: Produkt nach Beispiel 5
- Versuch 2: Produkt nach Beispiel 11, Schmelzbereich 40 bis 60 °C
- Versuch 3: Produkt nach Beispiel 11, Schmelzbereich 70 bis 90 °C.

**Patentansprüche**

1. Oligomere von Verbindungen der Formel

$$X\text{-}(CF_2)_a\text{-}O_b\text{-}(CH_2)_c\text{-}CH = CH_2 \qquad (1)$$

sowie Co-Oligomere aus Verbindungen der Formel (1) mit Verbindungen der Formel

$$X\text{-}(CX_2)_d\text{-}O_b\text{-}(CX_2)_c\text{-}CX = CX_2 \qquad (2) ,$$

in denen
X Wasserstoff oder Fluor,
a eine Zahl von 2 bis 16,
b und c - unabhängig voneinander - 0 oder 1 und d eine Zahl von 0 bis 6 bedeuten,
mit einem mittleren Oligomerisationsgrad von 2 bis 4.

2. Oligomere von Verbindungen der Formel (1) nach Anspruch 1, in denen X Fluor, a eine Zahl von 4 bis 12 und b und c Null bedeuten.

3. Verfahren zur Herstellung der Verbindungen nach Anspruch 1, dadurch gekennzeichnet, daß man eine Lösung von mindestens einer Verbindung der Formel (1), gegebenenfalls mit mindestens einer Verbindung der Formel (2), in einem Kohlenwasserstoff auf eine Temperatur von 135 bis 180 °C mit einem radikalbildenden Katalysator erhitzt.

4. Verwendung der Verbindungen nach Anspruch 1 als Schmier- oder Gleitmittel.

5. Verwendung der Verbindungen nach Anspruch 1 als Gleitmittel für Skisohlen.

6. Verwendung der Verbindungen nach Anspruch 1 als Hilfsmittel zur Applikation von Fluoralkanwachsen.

7. Verwendung der Verbindungen nach Anspruch 1 als Bestandteil von Skibelägen aus Polyethylen.

**Claims**

1. An oligomer of compounds of the formula

$$X\text{-}(CF_2)_a\text{-}O_b\text{-}(CH_2)_c\text{-}CH = CH_2 \qquad (1)$$

and co-oligomers of compounds of the formula (1) together with compounds of the formula

$$X\text{-}(CX_2)_d\text{-}O_b\text{-}(CX_2)_c\text{-}CX = CX_2 \qquad (2)$$

in which
X        is hydrogen or fluorine,
a        is a number from 2 to 16,
b and c  are, independently of one another, 0 or 1, and
d        is a number from 0 to 6,

7

with a mean degree of oligomerization of 2 to 4.

2. An oligomer of compounds of the formula (1) as claimed in claim 1, in which X is fluorine, a is a number from 4 to 12, and b and c are zero.

3. A process for preparing the compounds as claimed in claim 1, which comprises heating a solution of at least one compound of the formula (1), optionally with at least one compound of the formula (2), in a hydrocarbon to a temperature of 135 to 180°C together with a free radical-forming catalyst.

4. Use of the compounds as claimed in claim 1 as a grease or lubricant.

5. Use of the compounds as claimed in claim 1 as a lubricant for the underside of skis.

6. Use of the compounds as claimed in claim 1 as an aid for the application of fluoroalkane waxes.

7. Use of the compounds as claimed in claim 1 as a constituent of polyethylene ski coatings.

**Revendications**

1. Oligomères des composés de formule

$$X\text{-}(CF_2)_a\text{-}O_b\text{-}(CH_2)_c\text{-}CH=CH_2 \qquad (1)$$

ainsi que des co-oligomères de composés de formule (1) avec des composés de formule

$$X\text{-}(CX_2)_d\text{-}O_b\text{-}(CX_2)_c\text{-}CX=CX_2 \qquad (2),$$

dans lesquelles,
X signifie un hydrogène ou un fluor,
a signifie un nombre compris entre 2 et 16,
b et c - indépendamment l'un de l'autre - valent 0 ou 1 et
d est un nombre compris entre 0 et 6,
avec le degré d'oligomérisation moyen allant de 2 à 4.

2. Oligomères des composés de formule (1) selon la revendication 1, dans lesquels X signifie un fluor, a est un nombre allant de 4 à 12 et b et c valent 0.

3. Procédé de préparation des composés selon la revendication 1, caractérisé en ce qu'on chauffe une solution d'au moins un composé de formule (1), éventuellement avec au moins un composé de formule (2), dans un hydrocarbure à une température allant de 135 à 180°C avec un catalyseur générant des radicaux.

4. Utilisation des composés selon la revendication 1 en tant que lubrifiants ("Schmiermittel" et "Gleitmittel").

5. Utilisation des composés selon la revendication 1 en tant que lubrifiants pour semelles de skis.

6. Utilisation des composés selon la revendication 1 en tant qu'agents auxiliaires pour la préparation de cires à base de fluoroalcane.

7. Utilisation des composés selon la revendication 1 en tant que constituants de revêtements de skis à base de polyéthylène.